# EUROPEAN PATENT SPECIFICATION

(11) **EP 4 035 691 B1**
(45) Date of publication and mention of the grant of the patent: **15.11.2023**
(21) Application number: 22153821.8
(22) Date of filing: 28.01.2022
(51) Int. Cl.: A61L 2/00, A61L 2/10, A61L 2/24, A61L 9/20

(54) **INACTIVATION APPARATUS AND INACTIVATION METHOD**
INAKTIVIERUNGSVORRICHTUNG UND INAKTIVIERUNGSVERFAHREN
APPAREIL ET PROCÉDÉ D'INACTIVATION

(30) Priority: 02.02.2021 JP 2021014891
(43) Date of publication of application: 03.08.2022
(73) Proprietor: Ushio Denki Kabushiki Kaisha, Tokyo 100-8150 (JP)
(72) Inventor: IGARASHI, Tatsushi, Tokyo, 100-8150 (JP); OKUMURA, Yoshihiko, Tokyo, 100-8150 (JP)
(74) Representative: Maiwald GmbH

(56) References cited:
- WO-A1-2019/077817
- JP-A- 2017 528 258
- KR-A- 20200 135 849
- US-A1- 2020 282 087

## Description

### TECHNICAL FIELD

The present invention relates to an inactivation apparatus and an inactivation method for inactivating harmful microorganisms and/or viruses.

### BACKGROUND ART

Medical facilities, schools, government offices, theaters, hotels, restaurants, and other facilities where people frequently gather, or enter and leave are environments in which microorganisms such as bacteria, molds, or the like tend to proliferate and also viruses tend to spread. This tendency is in particular pronounced in confined or narrow spaces (i.e., enclosed spaces such as hospital rooms, toilet rooms, and inside elevators) and spaces where people are crowded together in the above facilities.

For example, harmful and highly infectious microorganisms and/or viruses are likely to proliferate on the floor, walls, and other surfaces of a certain space in a facility when a person infected with the virus enters and leaves the space, or they are likely to float in the space. As a result, the virus may infect the next person who enters the space, and in some cases, the infectious disease may spread within the facility.

In order to resolve such adverse situation described above, measures to disinfect harmful microorganisms (e.g., infectious microorganisms) or inactivate viruses as described above are required in facilities where humans (or animals, as the case may be) gather, or enter and leave.

The surfaces surrounding the space, such as the floor and walls, are decontaminated by workers by spraying disinfectant such as alcohol, wiping with a cloth soaked with disinfectant, or irradiating the surfaces with germicidal ultraviolet light. For microorganisms and viruses floating in the space, for example, sterilization and inactivation by ultraviolet light irradiation is performed.

Patent Literature 1 (Published Japanese Translation of the PCT International Publication No.2017-528258 A) discloses, as a decontamination apparatus for decontaminating an air-sealed room, an apparatus that irradiates the space to be decontaminated with ultraviolet light (i.e., UV-C light) when a user is not present to sterilize the space.

The wavelength range of ultraviolet light used for decontamination (or disinfection) applications is, for example, 200 nm to 320 nm. The wavelength that is in particular effective for disinfection is near 260 nm, where DNA absorption is high. For this reason, low-pressure mercury lamps emitting ultraviolet light having a wavelength of 253.7 nm are often used as light sources for disinfection. In recent years, ultraviolet LEDs having a peak wavelength of 275 nm have also been adopted.

However, ultraviolet light in the wavelength range described above have adverse effects on humans and animals. For example, it causes erythema, cancer due to DNA damage in the skin, and eye damage (hyperemia (e.g., conjunctival inflammation), inflammation of the cornea, or the like). In particular, light having a wavelength in the UV-C region (i.e., 200 to 280 nm), which is normally absorbed by the ozone layer in the stratosphere and do not reach the earth's surface, is also emitted from the above light sources, resulting in serious adverse effects on humans and animals.

On the other hand, studies on the safety of 222 nm ultraviolet light to humans and animals have begun to be reported in recent years.

For example, a Non-Patent Literature 1 (Sachiko Kaidzu, et al., "Evaluation of acute corneal damage induced by 222-nm and 254-nm ultraviolet light in Sprague-Dawley rats", FREE RADICAL RESEARCH, 2019, VOL. 53, NO. 6, p. 611-617), discloses the result of the evaluation of acute corneal damage, in which the eyes of albino rats were irradiated with 222 nm and 254 nm ultraviolet light with an irradiation amount (dose) of 30 mJ/cm², 150 mJ/cm², and 600 mJ/cm², respectively. In the Non-Patent Literature 1, it is reported that, in the case of the wavelength of 254 nm, keratitis was observed at the irradiation amount of 150 nJ/cm² or higher, while in the case of the wavelength of 222 nm, no damage to the cornea was observed even at the irradiation amount of 600 mJ/cm². Furthermore, document US2020/282087A1 (LATIF ROHULLAH [US] ET AL) discloses an inactivation method of inactivating microorganisms and/or viruses by emitting light and an inactivation apparatus for carrying out such method.

### LISTING OF REFERENCES

### PATENT LITERATURE

PATENT LITERATURE 1: Published Japanese Translation of PCT International Application Publication No. 2018-517488 A

### NON-PATENT LITERATURE

NON-PATENT LITERATURE 1: Sachiko Kaidzu, et al., "Evaluation of acute corneal damage induced by 222-nm and 254-nm ultraviolet light in Sprague-Dawley rats", FREE RADICAL RESEARCH, 2019, VOL. 53, NO. 6, p. 611-617

### SUMMARY OF THE INVENTION

### PROBLEMS TO BE SOLVED BY THE INVENTION

According to ACGIH (American Conference of Governmental Industrial Hygienists) and JIS Z 8812 (Measurement Method for Harmful Ultraviolet Radiation), the allowable limit value (i.e., Threshold Limit Value: TLV) for the irradiation amount of the ultraviolet light to the human body per day (i.e., 8 hours) is specified for each wavelength.

The TLV value in the above ACGIH, JIS Z 8812 and other standards for human and animal safety is specified as the irradiation amount of the ultraviolet light (i.e., the dose amount of the ultraviolet radiation). In the above Non-Patent Literature 1, a research report on safety is also based on the effects of the ultraviolet light radiated on humans and animals, using the irradiation amount of the ultraviolet light as a variable.

Thus, it has been considered that the degree of influence of the ultraviolet light on humans depends on the wavelength and the irradiation amount of the ultraviolet light.

The present invention has been made in order to solve the above-mentioned problems and an object thereof is to provide an inactivation apparatus and an inactivation method that are capable of inactivating harmful microorganisms and viruses more efficiently as well as reducing adverse effects on human eyes so as to ensure eye safety.

### SOLUTION TO PROBLEMS

In order to solve the above mentioned problems, according to one aspect of the present invention, there is provided an inactivation apparatus for inactivating microorganisms and/or viruses by emitting light, as defined in claim 5.

As a result of intensive research by the present inventors of the present invention, it has been newly found that even if the irradiation amount of the ultraviolet light is suppressed, it still has adverse effects on human eyes.

More particularly, the present inventors of the present invention have found that the effect on human eyes of ultraviolet light having a wavelength of 200 nm to 235 nm, which is considered to be light with little adverse effect on human and animal cells, depends on the irradiance of the ultraviolet light (i.e., effective incident irradiance on the human eyes). Furthermore, the present inventors have also found that there is no adverse effect on the human eyes when the effective incident irradiance of the ultraviolet light on the human eyes is within a certain range of irradiance.

As described above, by controlling the light source such that the effective incident irradiance of the ultraviolet light on eyes of a human present in a space to be irradiated is within a certain irradiation range, it makes it possible to inactivate microorganisms and/or viruses in the above space more efficiently while ensuring the safety of the human eyes.

In the above inactivation apparatus, the irradiance range have an upper limit equal to or less than 3.5 *µ*W/cm². Also, the irradiance range may have a lower limit equal to or greater than 1 *µ*W/cm².

In this case, it makes it possible to appropriately inactivate microorganisms and/or viruses in the space while better ensuring the safety of the human eyes.

Furthermore, in the above inactivation apparatus, the light source may be configured to emit the ultraviolet light from above the human to a floor surface, and a lower limit of the irradiance range may be equal to the effective incident irradiance of the ultraviolet light at a height of the eye of the human when the effective incident irradiance of the ultraviolet light at the floor surface is the minimum irradiance required for inactivation at the floor surface.

In this case, it makes it possible to appropriately inactivate microorganisms and/or viruses attached to the floor surface while ensuring the safety of the human eyes.

Yet furthermore, in the above inactivation apparatus, the controller unit may control the light source such that an irradiation amount of the ultraviolet light irradiated onto a human in the space is between 7 mJ/cm² and 150 mJ/cm².

In this case, it makes it possible to appropriately inactivate microorganisms and/or viruses in the space while ensuring the safety of the human eyes.

Yet furthermore, in the above inactivation apparatus, the effective incident irradiance of the ultraviolet light at the eye of the human may be an irradiance calculated based on an incident angle of the ultraviolet light from the light source to the eye of the human.

In this case, it makes it possible to control the light source in consideration of whether the ultraviolet light emitted from the light source is incident vertically on the human eyes or at a predetermined angle to the vertical incidence so as to appropriately ensure the safety of the human eyes.

Yet furthermore, in the above inactivation apparatus, assuming that a reference body height of the human is set to a reference height from the floor surface, the effective incident irradiance of the ultraviolet light at the eye of the human may be equal to the effective incident irradiance at the reference height.

In this way, the respective body heights of individual humans are uniformly assumed to be a predetermined reference body height, and the light source is controlled such that the effective incident irradiance at the reference body height is within a certain irradiance range. Thus, it makes it possible to attain the easy and appropriate control.

For example, in the case in which the light source is configured to emit the ultraviolet light from above a human to the floor surface, it makes it possible to easily obtain the distance from the light source to the human eyes by subtracting the above reference height from the height from the floor surface to the light source. In this case, it makes it possible to easily calculate the effective incident irradiance of the ultraviolet light on the human eyes based on the obtained distance to the human eyes, the incident angle of the ultraviolet light emitted from the light source with respect to the horizontal, and luminous intensity of the ultraviolet light.

Yet furthermore, in the above inactivation apparatus, the light source may be at least any one of an excimer lamp, an LED, and a coherent light source.

Excimer lamps, LEDs, and coherent light sources are less sensitive to the vibration, the pressure change, and the temperature change as compared to low-pressure mercury lamps, which have been used as ultraviolet light sources in the conventional inactivation apparatuses. In other words, the irradiance of the emitted light is less likely to become unstable even when the excimer lamps, LEDs, and coherent light sources are subjected to the vibration, the change in atmospheric pressure, or the change in temperature. For this reason, by using the excimer lamps, LEDs, and coherent light sources as the light source, it makes it possible to stably emit light even when the inactivation apparatus is used in an environment subject to the vibration, the change in atmospheric pressure, or the change in temperature. As a result, it makes it possible to appropriately perform sterilization and inactivation.

Yet furthermore, in the above inactivation apparatus, the light source may emit ultraviolet light having a center wavelength of 222 nm.

In this case, it makes it possible to inactivate microorganisms and/or viruses more efficiently while appropriately suppressing the irradiated ultraviolet light from adversely affecting the human bodies.

Yet furthermore, in the above inactivation apparatus, the light source may be an LED, and the LED may be at least any one of an aluminum gallium nitride (AlGaN) based LED, an aluminum nitride (AIN) based LED, and a magnesium zinc oxide (MgZnO) based LED.

In this case, the LED, which is less sensitive to the vibration, the change in atmospheric pressure, and the change in temperature, is used to emit, for example, ultraviolet light having the wavelength range of 200 nm to 235 nm, which is less harmful to the human bodies. Thus, it makes it possible to inactivate microorganisms and/or viruses more stably and appropriately.

Yet furthermore, in the above inactivation apparatus, the light source may be an LED, and the light irradiation unit may include a cooling member to cool the LED.

In this case, it makes it possible to appropriately suppress the temperature of the LED from rising so as to emit light from the LED more stably.

Yet furthermore, in the above inactivation apparatus, the light source may be an excimer lamp, and the light irradiation unit may include a housing made of conductive metal and configured to house the excimer lamp.

In this case, it makes it possible to suppress the high frequency noises generated by the excimer lamp from being transmitted to the outside of the housing. As a result, it makes it possible to suppress the high frequency noises caused by the excimer lamp from adversely affecting control commands to the control system installed outside the housing so as to prevent defects in the control commands from occurring.

Yet furthermore, in the above inactivation apparatus, the light irradiation unit may include a housing configured to house the excimer lamp and equipped with a light emission window emitting at least a part of light emitted from the light source, and
the light emission window may be equipped with an optical filter configured to prevent light in a wavelength range other than 200 nm to 235 nm from passing through the optical filter.

In this case, it makes it possible to irradiate the space solely with light having the wavelength range that has minimal adverse effects on the human bodies and animals.

According to another aspect of the present invention, there is provided an inactivation method of inactivating microorganisms and/or viruses by emitting light, as defined in claim 1.

In this way, by controlling the light source such that the effective incident irradiance of the ultraviolet lightatthe eyes of a human existing in the space is within a certain irradiance range, it makes it possible to inactivate microorganisms and/or viruses in the above space while ensuring the safety of human eyes.

### ADVANTAGEOUS EFFECT OF THE INVENTION

According to the present invention, it makes it possible to inactivate harmful microorganisms and/or viruses more efficiently, and also to reduce the adverse effects on the human eyes, thereby ensuring the safety of human eyes.

The above mentioned and other not explicitly mentioned objects, aspects and advantages of the present invention will become apparent to those skilled in the art from the following embodiments (detailed description) of the invention by referring to the accompanying drawings and the appended claims.

### BRIEF DESCRIPTION OF DRAWINGS

FIG. 1 is a chart exemplarily illustrating the UV absorption spectrum of protein.
FIG. 2 is a schematic diagram exemplarily illustrating an experimental system for evaluation experiments on human eyes.
FIG. 3 is a chart exemplarily illustrating the spectral distribution of the light transmittance in an example of an optical filter.
FIG. 4 is a table showing results of the evaluation experiments.
FIG. 5 is a schematic diagram illustrating an exemplary configuration of an inactivation system provided with an inactivation apparatus according to the present embodiment.
FIG. 6 is a schematic diagram illustrating an exemplary configuration of an ultraviolet light (UV) irradiation unit.
FIG. 7A is a schematic diagram illustrating an exemplary configuration of an excimer lamp.
FIG. 7B is a schematic diagram illustrating an exemplary configuration of an excimer lamp.
FIG. 8A is a schematic diagram illustrating another exemplary configuration of the excimer lamp.
FIG. 8B is a schematic diagram illustrating another exemplary configuration of the excimer lamp.
FIG. 9A is a schematic diagram illustrating yet another exemplary configuration of the excimer lamp.
FIG. 9B is a schematic diagram illustrating yet another exemplary configuration of the excimer lamp.
FIG. 10 is a schematic diagram illustrating another exemplary configuration of the UV irradiation unit.

### DESCRIPTION OF EMBODIMENTS

Hereinafter, non-limiting embodiments of the present invention will be described in detail with reference to the accompanying drawings. Among the constituent elements disclosed herein, those having the same function are denoted by the same reference numerals, and a description thereof is omitted. It should be noted that the embodiments disclosed herein are illustrative examples as means for implementing the present invention, and should be appropriately modified or changed depending on a configuration and various conditions of an apparatus to which the present invention is applied, and the present invention is not limited to the following embodiments. Furthermore, it should be noted that all of the combinations of features described in the following embodiments are not necessarily essential to the solution of the present invention.

The present embodiment will describe an inactivation system that performs ultraviolet light (hereinafter referred to as "UV light" or simply referred to as "UV") irradiation in a space where humans are present and inactivates microorganisms and/or viruses existing in the space.

It should be noted that the term "inactivation" in the present embodiment refers to the death of microorganisms and/or viruses (or the loss of infectivity and/or toxicity). Also, it should be noted that the term "space where a human is present" is not limited to spaces where a human is actually present but includes spaces where a human can enter and leave but no human is present.

Here, the above spaces may be, for example, spaces in facilities such as offices, commercial facilities, medical facilities, station facilities, schools, governmental facilities, theaters, hotels, restaurants, or the like. The above spaces may also include spaces in vehicles such as automobiles, trains, buses, cabs, airplanes, ships, or the like. It should be noted that the above spaces may be enclosed, semi-enclosed spaces such as hospital rooms, conference rooms, restrooms, or inside elevators, or otherwise unclosed spaces.

The inactivation apparatus according to the present embodiment irradiates an area where a human is present with UV light having a wavelength of 200 nm to 235 nm, which has little adverse effect on the cells of humans and animals to inactivate harmful microorganisms and/or viruses that exist on a surface of objects or spaces in the area. Here, the above objects include human bodies, animals, and other objects.

For practical purposes, the wavelength range of UV light used for decontamination (or sterilization) is assumed to be between 200 nm to 320 nm. In particular, it is common to use UV light having a wavelength near 260 nm, where the absorption of nucleic acids (DNA, RNA) possessed by microorganisms and/or viruses is high. However, such UV light having the wavelength range near 260 nm adversely affects humans and animals. For example, the UV light having the wavelength range near 260 nm causes cancer due to erythema or DNA damage in the skin, as well as eye damage (hyperemia (conjunctivitis), inflammation of the cornea, etc.).

For this reason, conventional UV irradiation systems, which use UV light having the wavelength near 260 nm for decontamination (sterilization) as described above, are configured to emit UV light when no humans or animals are present, and to stop emitting UV light when humans or animals are present in the area to be irradiated, in consideration of the safety of humans or animals.

However, it is also observed that the propagation of harmful microorganisms in a space, the floating of microorganisms and/or viruses, and their attachment to surfaces surrounding the space are often caused by the entry and exit of humans (i.e., infected persons) or animals carrying harmful microorganisms and/or viruses. Therefore, it is essentially efficient for a UV irradiation system for decontamination (disinfection) to decontaminate not only the space and surfaces surrounding the space, but also the surfaces of humans or animals in the space.

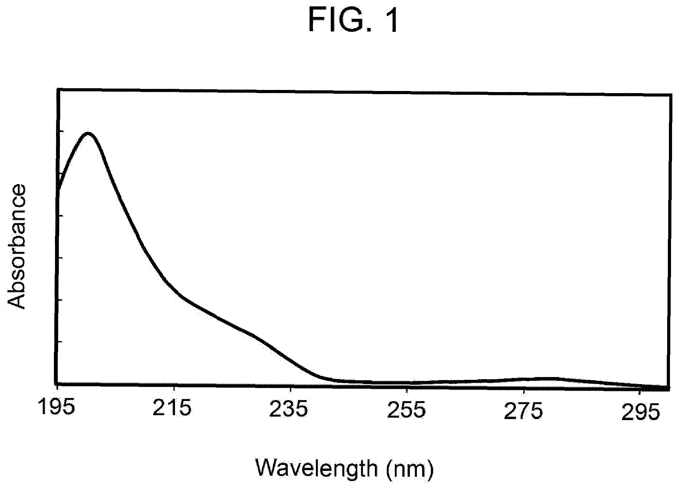

FIG. 1 is a chart exemplarily illustrating the UV absorption spectrum of protein.

As shown in FIG. 1, it can be observed that the protein has a light absorption peak at a wavelength of 200 nm, while UV light having a wavelength of 240 nm or longer is unlikely to be absorbed. This means that the UV lights having the wavelength of 240 nm or longer is likely to pass through human skins and further penetrate skin inner tissues. As a result, cells inside the human skin are likely to be damaged. On the other hand, the UV light having the wavelength near 200 nm is absorbed by surfaces of the human skins (e.g., stratum corneum) and do not penetrate the skin inner tissues. Therefore, the UV light having the wavelength near 200 nm is safe for the skin.

On the other hand, the UV light having a wavelength less than 200 nm may produce ozone (O₃). This is because, when the UV light having the wavelength less than 200 nm is emitted in an atmosphere containing oxygen, oxygen molecules are photolyzed to produce oxygen atoms, and ozone is produced by the bonding reaction between oxygen molecules and oxygen atoms.

Therefore, the wavelength range of 200 nm to 240 nm is safe for humans and animals. Furthermore, the wavelength range that is safe for humans and animals is preferably 200 nm to 237 nm, more preferably 200 nm to 235 nm, and even more preferably 200 nm to 230 nm.

The inactivation apparatus according to the present embodiment uses the UV light having the wavelength range of 200 nm to 235 nm, which is a safe wavelength range for humans and animals, and irradiates a space where a human is present with the UV light, instead of preventing humans from being irradiated with the UV light, as in the conventional systems.

It should be noted that according to the ACGIH (American Conference of Government Industrial Hygienists) and JIS Z 8812 (Measurement method of harmful ultraviolet radiation), the allowable limit value (TLV: Threshold Limit Value) for the irradiation amount of UV light to the human body per day (i.e., 8 hours) is specified for each wavelength.

The underlying consideration behind the above safety standard is that if a human is exposed to UV light with the irradiation amount that exceeds the TLV value, then the UV irradiation will lead to adverse effects in a part of human body exposed to the UV irradiation.

This implies that the greater the irradiation amount of the UV light, the greater the degree of adverse effects on humans. For example, when a body part irradiated with the UV light is the skin, the degree of influence of the skin cancer is taken into account. Likewise, when a body part irradiated with the UV light is the eye, the degree of influence of keratitis is taken into account.

As described above, it has been considered that the degree of influence of the UV light on humans depends on the wavelength and the irradiation amount (i.e., dose) of the UV light. However, it has been newly found that even when the irradiation amount of UV light is suppressed, it still has adverse effects on the human eyes.

As a result of the present inventors' intensive research, it has been found that, for the human eyes, apart from the acute damage caused solely by the irradiation amount of the UV light, there is another symptom that depends on the irradiance (in other words, light intensity) of the UV light.

An acute disorder of the eye that is affected by the irradiation amount (i.e., dose) of the UV light is, for example, ultraviolet keratitis. Keratitis is the eye disorder in which, as the irradiation amount of the UV light increases, the amount and depth of damage to the corneal epithelium increases, and several layers of the corneal epithelium undergo apoptosis and fall off, causing the eye to feel like it has sand in it. Subsequently, the nerve underneath the corneal epithelium is exposed on the surface, and for two to three days, until a new corneal epithelium is formed, the disorder causes pain that keeps the patient awake at night. The irradiation amount of the UV light that causes keratitis is assumed to be 600 mJ/cm² or more according to the experiments with mice irradiated with the UV light having a wavelength of 222 nm.

On the other hand, the present inventors have found that even lower irradiation amount of the UV light may cause undesirable symptom in daily life, although they are insufficient to be described as disorders. For example, a considerable amount of tears and discomfort have been observed for a certain period of time after the UV irradiation.

To cope with those symptoms, the present inventors irradiated the human eyes with the UV light and evaluated the effects thereof. This kind of evaluation on the human eyes (especially the evaluation of the effects on the human eyes of the UV light having the wavelength of 200 nm to 240 nm, which is considered safe for humans, as described above) has not been conducted since the 1970s. There was only one case of a wavelength-specific test for the UV light having this wavelength range in the 1970s, but no further tests were conducted. The present inventors were the first to conduct a detailed evaluation of the UV light having the wavelength of 200 nm to 240 nm.

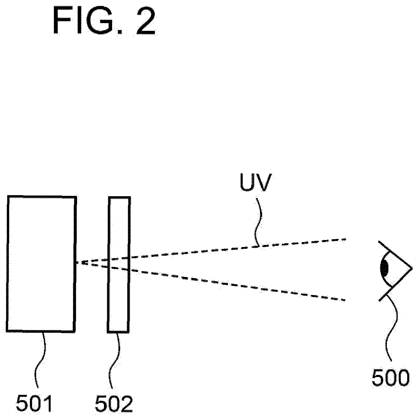

FIG. 2 is a schematic diagram exemplarily illustrating an experimental system for the evaluation experiment on the human eyes.

As shown in FIG. 2, ultraviolet (UV) light emitted from a light source device 501 were incident vertically on human eyes 500, and the condition of the eye 500 after being irradiated with the UV light was examined.

As the light source device 501, the experiment used a KrCI excimer lamp light source device, which is equipped with a KrCl excimer lamp having a center wavelength of 222 nm and emits light from the KrCI excimer lamp to the outside.

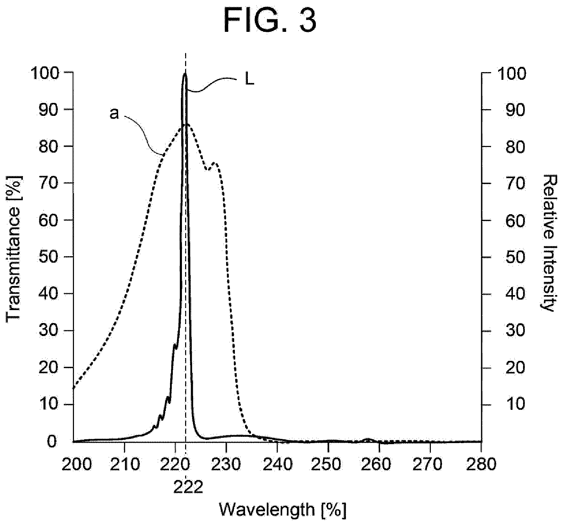

It should be noted that KrCI excimer lamps also emit UV light having the wavelength range longer than 240 nm, which adversely affects humans and animals, as shown by the spectrum in the solid line L in FIG. 3. For this reason, in the above experiment, an optical filter 502, which blocks the UV light (UV-C) having the wavelength range longer than 235 nm, was arranged between the KrCI excimer lamp light source device 501 and the eye 500. The dashed line a in FIG. 3 shows the spectral transmittance characteristics of the optical filter 502 used in the above experiment.

The optical filter 502 in the above experiment is composed of a dielectric multilayer film, which is formed by alternating layers of HfO₂ layer and SiO₂ layer on one side of a substrate made of synthetic quartz glass. For example, the thickness of the HfO₂ layer in the dielectric multilayer film is approximately 240 nm, and the thickness of the SiO₂ layer is approximately 1460 nm, respectively, and the total number of layers of the HfO₂ and SiO₂ layers is 33 layers. An anti-reflection (AR) coating with HfO₂ and SiO₂ layers is applied to the other side of the substrate of the optical filter 502.

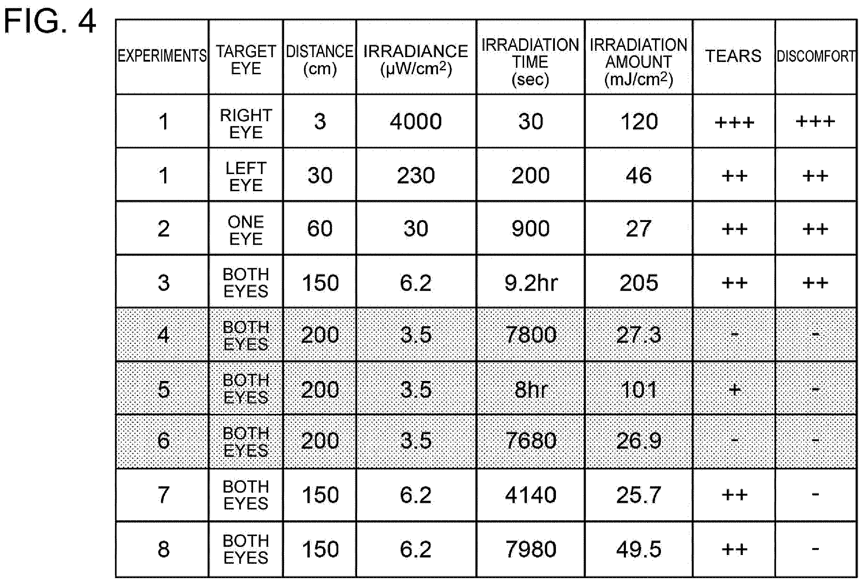

FIG. 4 is a table showing the experimental results of the evaluation experiments on the human eyes.

As shown in FIG. 4, eight experiments were conducted in which one or both eyes were irradiated with the UV light described above (Experiments 1 to 8).

The irradiation amounts (doses) of the UV light vary from 25.7 mJ/cm² to 205 mJ/cm², and the irradiance on the ocular surface and the irradiation time in each experiment are shown in the table in FIG. 4.

All of the experiments were conducted on human eyes (i.e., human ocular globes). The adverse effects of irradiation with the UV light (that is, the KrCI excimer lamp light having the center wavelength of 222 nm, with UV light above 235 nm being cut off by the optical filter 502) on the human eyes were judged by whether tears were noticeably produced or there was discomfort to the eyes after 10 to 120 minutes (i.e., 2 hours) from the UV irradiation.

In the "Tears" column of FIG. 4, "-" denotes no tears,"+" denotes a small amount of tears,"+ +" denotes a large amount of tears, and "+ + +" denotes a larger amount of tears. In the "Discomfort" column of FIG. 4, "-" denotes no discomfort, followed by "+", "++", and "+++" for larger discomfort in this order.

As described above, it has been conventionally considered that the greater the irradiation amount of UV light, the greater the degree of adverse effects on humans.

However, as shown in FIG. 4, it has been turned out that there is not always a positive correlation between the irradiation amount of UV light and the degree of tears or discomfort, or the like.

For example, comparing the results between the right eye of the Experiment 1 and both eyes of the Experiment 3, the UV irradiation amounts were 205 mJ/cm² in the Experiment 3 and 120 mJ/cm² in the Experiment 1, respectively. It means that the UV irradiation amount in the Experiment 3 is about 1.7 times higher than that in the Experiment 1. Nevertheless, it is observed that the adverse effect on the human eyes, such as tears and discomfort, is smaller in the Experiment 3 than in the Experiment 1.

Likewise, comparing the results among the cases of one eye in the Experiment 2, both eyes in the Experiment 4, both eyes in the Experiment 6, and both eyes in the Experiment 7, the UV irradiation amounts were 25.7 mJ/cm² to 27.3 mJ/cm², which are approximately same among those experiments. Nevertheless, the degree of adverse effect on the human eye was as follows: no adverse effect in the Experiment 4 and the Experiment 6, tears produced but no discomfort in the Experiment 7, and both of tears produced and discomfort to some extent greater in the Experiment 2.

It has been turned out that, as shown in those results above, the degree of adverse effect by the UV light at least on the human eyes does not necessarily increase with the increase in the irradiation amount of UV light.

Furthermore, comparing the results of the right eye of the Experiment 1 and both eyes of the Experiment 3 in terms of the irradiance on the surface of the human eye, the irradiance on the surface of the eye was 6.2*µ* W/cm² in the Experiment 3, and 4000 *µ*W/cm² in the Experiment 1, respectively. It means that the irradiance in the Experiment 1 is about 645 times higher than that in the Experiment 3. Also, the adverse effect on the human eye in the Experiment 1 is greater than that in the Experiment 3.

Likewise, comparing the results of one eye in the Experiment 2, both eyes in the Experiment 4, both eyes in the Experiment 6, and both eyes in the Experiment 7 in terms of irradiance on the surface of the eye, the irradiance of the UV light was, in ascending order, 3.5 *µ*W/cm² in the Experiment 4 and the Experiment 6, 6.2 *µ*W/cm² in the Experiment 7, and 30 *µ*W/cm² in the Experiment 2. The degree of the effects on the human eye is also, in ascending order, the Experiments 4 and 6, the Experiment 7, and the Experiment 2.

As a result, for the first time, it has been turned out that the degree of adverse effect at least on the human eye increases with the increase in the irradiance of the UV light on the human eye.

Furthermore, when the irradiance on the human eye is the same, it has been turned out that the larger the irradiation amount of the UV light, the greater the adverse effect on the human eye.

For example, comparing the results of both eyes of the Experiments 3, 7, and 8, the irradiance at the eye was the same, 6.2 *µ*W/cm², which is relatively small. However, the adverse effect on the human eye was greater in the case of the Experiment 3, where the UV irradiation amount was 205 mJ/cm², than in the Experiment 7, where the UV irradiation amount was 25.7 mJ/cm², and in the Experiment 8, where the UV irradiation amount was 49.5 mJ/cm².

The above findings from the experimental results shown in FIG. 4 indicate that when the irradiance of the UV light on the human eye is equal to or less than 3.5 *µ*W/cm², the production (i.e., secretion) of tears in the human eye is small, if any, and does not cause any discomfort.

Based on those findings, the present inventors further examined the adverse effects of the UV irradiation on the human eye when the irradiance of the UV light was 3.5 *µ*W/cm² and the irradiation amount of the UV light was 150 mJ/cm². Even in this case, the production of tears in the human eye was small and no discomfort occurred.

It should be noted that the irradiance of the UV light at the human eye is preferably equal to or greater than 1 *µ*W/cm² in order to ensure the virus inactivation.

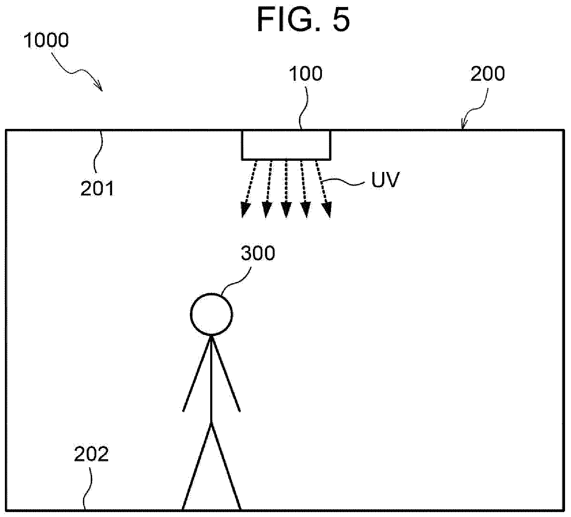

More particularly, the minimum UV irradiation amount required to inactivate 90% of viruses (e.g., new coronaviruses) is 0.6 mJ/cm². For example, as shown in FIG. 5, consider an inactivation system 1000 that emits UV light downwardly toward a floor surface 202 in which an inactivation apparatus 100 that emits UV light having the center wavelength of 222 nm is installed on a ceiling 201 of a certain facility 200, which is a space where a human 300 is present. Assuming that the height from the floor surface 202 to the ceiling 201 (i.e., the light-emitting surface of the inactivation apparatus 100) is 2.5 m, and the height from the floor surface 202 to the eyes of the human 300 is 1.7 m, the irradiance at the eye level of the human 300 is approximately 11 times of the irradiance at the floor surface 202. Thus, when the irradiation amount of the UV light at the eye level of the human 300 is 6.6 mJ/cm², the irradiation amount of the UV light at the floor surface 202 is 0.6 mJ/cm².

When the irradiance of the UV light on the human eye is 1 *µ*W/cm², the irradiation amount of the UV light at the height of the human eye reaches 6.6 mJ/cm² with the irradiation time of 110 minutes. In other words, when the irradiance of the UV light on the human eye is 1 *µ*W/cm², it is possible to inactivate the viruses on the floor surface 202 with an irradiation time of less than 2 hours, which is highly practical. In this way, it makes it possible to ensure the safety of the human eyes and also achieve the virus inactivation, by setting the lower limit of the irradiance range of the irradiance on the human eye to the irradiance at the height of the human eye when the irradiance of the UV light at the floor surface 202 becomes the minimum irradiance necessary for virus inactivation at the floor surface 202.

To sum up, it has been found that when the irradiance of the UV light on the human eye is between 1 *µ*W/cm² and 3.5 *µ*W/cm², the production of tears in the human eye is negligibly small, if any, and does not cause any discomfort.

It should be noted that the term "irradiance on the human eye" in the present disclosure refers to the effective incident irradiance on the human eye (i.e., the effective value of the irradiance of the light incident on the human eye), which is the irradiance calculated based on the incident angle of the UV light from the UV light source to the human eye. When the UV light is incident on the human eye at an angle of 30° with respect to the vertical incidence, the effective incident irradiance can be calculated by multiplying the irradiance at the vertical incidence by cos 30° = 0.86.

Furthermore, the irradiance of the UV light at the human eye is not limited to the value actually measured by a sensor or the like at the position of the human eye, but may also be a value obtained by calculation. For example, as shown in FIG. 5, when the UV light source is configured to emit UV light from above the human 300 to the floor surface 202, assuming that the height of any human is uniformly a predetermined reference height (e.g., 180 cm), the distance from the UV light source to an object to be irradiated (e.g., human eye) can be obtained by subtracting the value of the above reference body height (i.e., reference height) from the height of the installed UV light source from the floor. In this case, the irradiance on the human eye can be calculated based on the obtained distance and the luminous intensity of the UV light emitted from the UV light source. It should be noted that the irradiance on the human eye can be calculated using the height of the human eye (e.g., 170 cm), which is derived based on the above reference body height, as the above reference height.

The reason why the tear production and discomfort do not occur when the irradiance is 3.5 *µ*W/cm² or less, even at the same UV irradiation amount can be considered, although not necessarily apparent, as follows.

The UV light having the wavelength of 222 nm is absorbed by the first layer of corneal epithelium in the eye and does not penetrate any deeper. The first layer of the corneal epithelium, which has absorbed the 222 nm UV light, is damaged by the 222 nm UV light to some extent. However, the damaged part in the first layer of the corneal epithelium then disappears naturally through the turnover (i.e., metabolic turnover). The rate (speed) of this turnover is approximately 10 hours.

Resultantly, provided that the rate (speed) of apoptosis in the first layer of the corneal epithelium, which is caused by damage from the UV light having the wavelength of 222 nm, does not exceed the rate of the metabolic turnover in the first layer of the corneal epithelium, it can be assumed that damage will not occur in the second layer and any deeper of the corneal epithelium and thus the tear production and discomfort will not occur.

In other words, the present inventors have found for the first time that the apoptosis rate of the first layer of the corneal epithelium does not exceed the turnover rate when the human eye is irradiated with the UV light at the irradiance equal to or less than 3.5 *µ*W/cm².

As described above, it has been found that, in the inactivation apparatus that performs sterilization and inactivation with UV light having the wavelength of 200 nm to 235 nm, which are considered safe for humans, as long as the irradiance on the human eye is equal to or less than 3.5 *µ*W/cm², the UV light will not cause any symptoms such as eye discomfort or excessive tear production even if the human looks into the UV light source and the human eye is exposed to the UV light source for a relatively long time. It enhances the degree of freedom in arranging the inactivation apparatus for sterilization and inactivation without considering the problem of incidence on the human eye.

It is preferable that the irradiation amount of the UV light on the human eye is between 7 mJ/cm² and 150 mJ/cm².

As described above, in the inactivation system 1000 shown in FIG. 5, in order to ensure that the irradiation amount of the UV light at the floor surface 202 is to be the minimum irradiation amount required to inactivate 90% of viruses (i.e, 0.6 mJ/cm²), the UV irradiation amount of 6.6 mJ/cm² is required at the height of the eye of the human 300. Therefore, it is preferable to set the irradiation amount of the UV light on the human eye to be equal to or greater than 7 mJ/cm².

On the other hand, in the case of the irradiance on the human eye is 3.5 *µ*W/cm², the UV irradiation amount is 100 mJ/cm² when irradiated for 8 hours per day, and the UV irradiation amount is 150 mJ/cm² when irradiated for 12 hours per day. Although the TLV values according to the safety standard for humans and animals such as the ACGIH and JIS Z 8812 are specified as the UV irradiation amount (UV dose) for 8 hours per day, it is preferable to set the UV irradiation amount on the human eye to be equal to or less than 150 mJ/cm², in consideration of the case where UV irradiation for 12 hours is operationally required.

As described above, the inactivation apparatus 100 according to the present embodiment emits UV light having the wavelength range of 200 nm to 235 nm in a space where a human is present to inactivate microorganisms and/or viruses existing in the space. According to the present embodiment, the inactivation apparatus 100 controls the UV light source such that the effective incident irradiance of the UV light on the eyes of a human present in the space is within a certain irradiance range. More particularly, the inactivation apparatus 100 controls the UV light source such that the effective incident irradiance of the UV light on the eye of a human present in the space is between 1 *µ*W/cm² and 3.5 *µ*W/cm².

In addition, the inactivation apparatus 100 may control the UV light source such that the irradiation amount of UV light irradiated to a human in the space is between 7 mJ/cm² and 150 mJ/cm².

It makes it possible to appropriately inactivate microorganisms and/or viruses in the above space while ensuring the safety of human eyes, even in situations where UV light may be incident on human eyes in a space where a human is present.

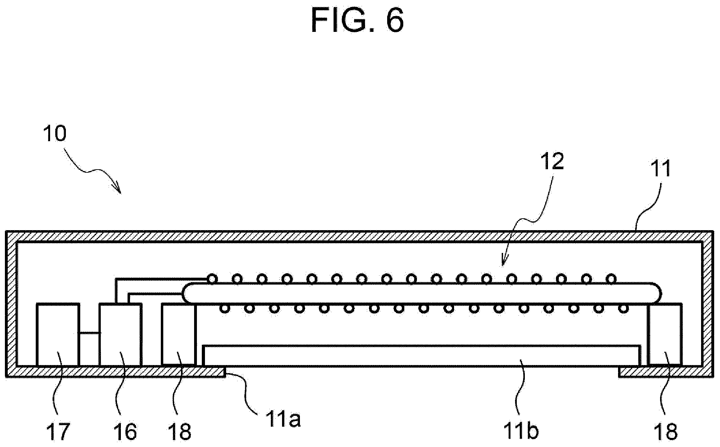

FIG. 6 is a schematic diagram exemplarily illustrating the inactivation apparatus 100 described above.

In FIG. 6, the UV irradiation unit 10 is mainly shown, which is a part relating to the UV irradiation.

The UV irradiation unit 10 is equipped with a housing 11 made of conductive metal and a UV light source 12 housed inside the housing 11.

The UV light source 12 may be, for example, a KrCI excimer lamp that emits UV light having the center wavelength of 222 nm. It should be noted that the UV light source 12 is not limited to KrCI excimer lamps, but may be any light source that emits UV light having the wavelength range of 200 nm to 235 nm.

The UV irradiation unit 10 is also equipped with a power supply unit 16 that supplies power to the excimer lamp 12, and a controller unit 17 that controls the irradiation and non-irradiation of the excimer lamp 12 and the light intensity of the UV light emitted from the excimer lamp 12.

The excimer lamp 12 is supported by the support member 18 in the housing 11.

The housing 11 has an opening 11a that serves as a light emission window. A window member 11b is provided in the opening 11a. The window member 11b may include, for example, a UV transmitting member made of quartz glass and an optical filter that blocks unnecessary light.

A plurality of excimer lamps 12 may be arranged in the housing 11. The number of excimer lamps 12 is not particularly limited.

As the above optical filter, for example, a wavelength selective filter that transmits light in the wavelength range of 200 nm to 235 nm and blocks (cuts off) other light in the UV-C wavelength range (i.e., light in the wavelength range of 236 nm to 280 nm) may be used.

As the wavelength selective filter, for example, the dielectric multilayer filter with HfO₂ and SiO₂ layers shown in FIG. 3 may be used.

Alternatively, an optical filter with a dielectric multilayer of SiO₂ and Al₂O₃ layers may be used as the wavelength selective filter.

In this way, by arranging the optical filter on the light emission window, even when the excimer lamp 12 emits light that is harmful to humans, it makes it possible to more reliably suppress the light from leaking to the outside of the housing 11.

Hereinafter, the exemplary configuration of the excimer lamp 12, which is used as the UV light source in the UV irradiation unit 10, will be described in detail.

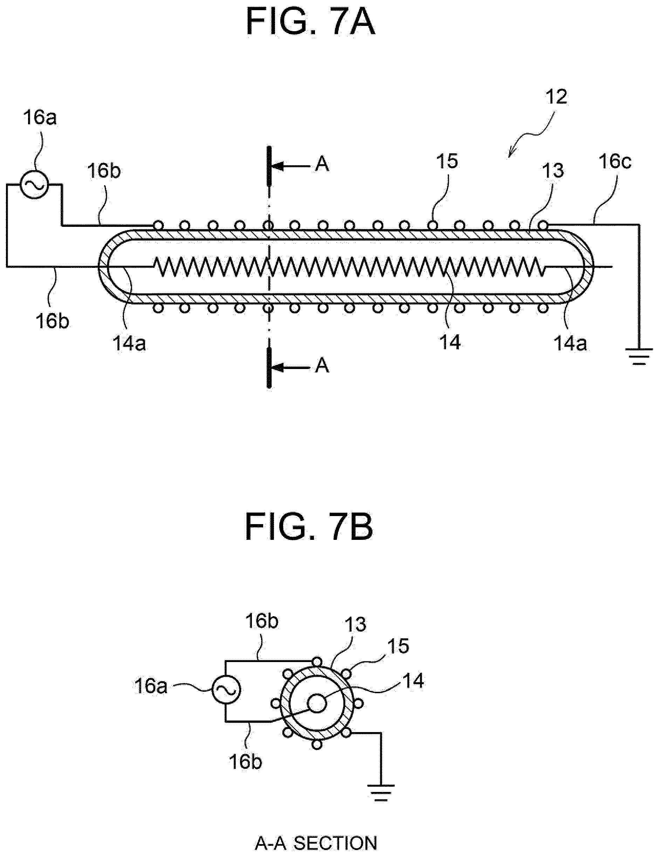

FIG. 7A is a schematic diagram of the cross section of the excimer lamp 12 in the direction of the tube axis, and FIG. 7B is the A-A cross sectional view of FIG. 7A.

As shown in FIGs. 7A and 7B, the excimer lamp 12 is equipped with a long rectangular tube-shaped discharge vessel 13 with both ends thereof air-tightly sealed. The discharge vessel 13 is made of a dielectric material having light transmittance that transmits UV light, such as synthetic quartz glass or fused silica glass. A discharge space is formed inside the discharge vessel 13, where a rare gas and a halogen gas are enclosed as a barrier discharge gas that produces UV light (hereinafter referred to as "discharge gas"). According to the present embodiment, krypton (Kr) is used as the rare gas and chlorine gas (Cl₂) is used as the halogen gas.

Alternatively, a mixture of krypton (Kr) and bromine (Br₂) may be used as the discharge gas. In this case, the excimer lamp (i.e., KrBr excimer lamp) emits UV light having a center wavelength of 207 nm.

A first electrode (i.e., inner electrode) 14 is arranged in the discharge space inside the discharge vessel 13. The inner electrode 14 is a coil-shaped electrode, which is formed by winding metal strands made of electrically conductive and heat-resistant metal, such as tungsten, into a coil with a coil diameter smaller than the inner diameter of the discharge vessel 13. The inner electrode 14 extends along the central axis (i.e., tube axis) of the discharge vessel 13 and is arranged such that the inner electrode 14 does not contact the inner surface of the discharge vessel 13.

Each of the two ends of the inner electrode 14 is electrically connected to one end of each of the lead members 14a for the inner electrode 14. Each of the other ends of the lead members 14a for the inner electrode 14 protrudes outward from the outer end surface of the discharge vessel 13.

A second electrode (i.e., outer electrode) 15 is arranged on the outer periphery of the discharge vessel 13. The outer electrode 15 is a reticular (net-like) electrode composed of metal strands made of electrically conductive and heat-resistant metal, such as tungsten. The outer electrode 15 is provided such that the outer electrode 15 extends in the direction of the central axis of the discharge vessel 13 along the outer periphery of the discharge vessel 13. In the excimer lamp 12 shown in FIGs. 7A and 7B, the outer electrode 15, which is a reticular electrode, has a cylindrical outer shape and is provided in close contact with the outer periphery of the discharge vessel 13.

With the above configuration, the discharge region is formed in a region where the inner electrode 14 and outer electrode 15 face each other through the tube wall (i.e., dielectric material wall) of the discharge vessel 13 inside the discharge space.

Furthermore, one end of the outer electrode 15 and the other end of one of the lead members 14a for the inner electrode 14 are connected to a high-frequency power supply 16a provided by the power supply unit 16 (see FIG. 6) via a power feed line 16b, respectively. The high-frequency power supply 16a is a power supply capable of applying a high-frequency voltage between the inner electrode 14 and the outer electrode 15.

One end of the lead wire 16c is electrically connected to the other end of the outer electrode 15, and the other end of the lead wire 16c is grounded. In other words, the outer electrode 15 is grounded via the lead wire 16c. In the excimer lamp 12 shown in FIGs. 7A and 7B, one of the lead members 14a for the inner electrode 14 is integrated with the power feed wire 16b.

When high-frequency power is applied between the inner electrode 14 and the outer electrode 15, a dielectric barrier discharge is generated in the discharge space. This dielectric barrier discharge excites the atoms of the discharge gas (i.e., barrier discharge gas) enclosed in the discharge space and produces an excited dimer (i.e., exciplex). When those excited dimers return to their original state (i.e., ground state), an inherent luminescence (i.e., excimer luminescence) is produced. In other words, the above discharge gas is an excimer emission gas.

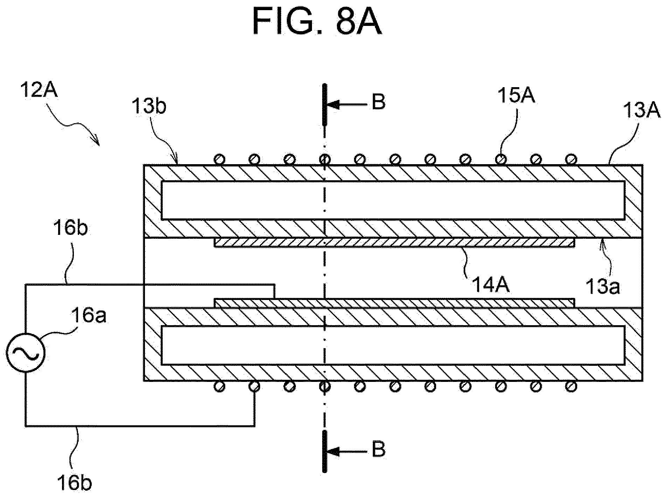

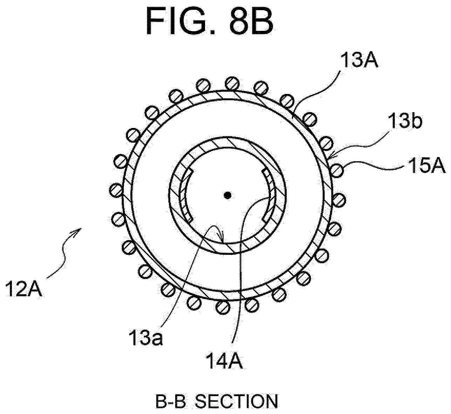

It should be noted that the configuration of excimer lamps is not limited to that shown in FIGs. 7A and 7B. For example, as shown in FIGs. 8A and 8B, the excimer lamp 12A may be equipped with a double-tube discharge vessel 13A.

The discharge vessel 13A of the excimer lamp 12A has a cylindrical outer tube and a cylindrical inner tube that is coaxially arranged inside the outer tube and has a smaller inner diameter than the outer tube. The outer tube and the inner tube are sealed at the left and right ends as shown in FIG.8A, and a circular inner space is formed therebetween. The discharge gas is enclosed in this inner space.

A reticular first electrode (i.e., inner electrode) 14A is arranged on the inner wall 13a of the inner tube, and a reticular or mesh-like second electrode (i.e., outer electrode) 15A is arranged on the outer wall 13b of the outer tube.

The inner electrode 14A and the outer electrode 15A are electrically connected to the high-frequency power supply 16A via the power feed line 16. The inner electrode 14A and the outer electrode 15A are electrically connected to the high-frequency power supply 16A via the feed wire 16B, respectively.

A high-frequency AC voltage is applied by the high-frequency power supply 16a between the inner electrode 14A and the outer electrode 15A, which causes a voltage to be applied to the discharge gas through the outer and inner tubes, and a dielectric barrier discharge is generated in the discharge space where the discharge gas is enclosed. This excites the atoms of the discharge gas to produce an excited dimer, and when these atoms shift to the ground state, excimer emission is produced.

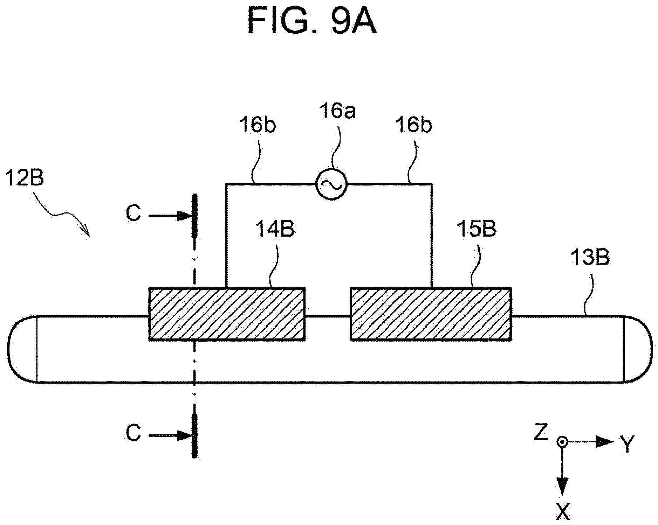

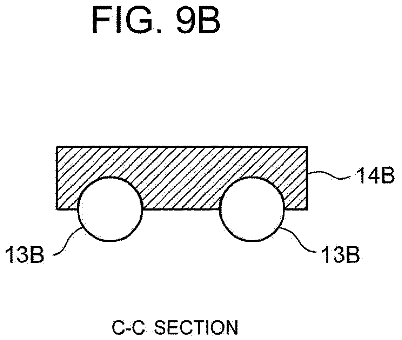

Alternatively, the excimer lamp may have the configuration of, for example, an excimer lamp 12B as shown in FIGs. 9A and 9B in which a pair of electrodes (i.e., first electrode 14B and second electrode 15B) are arranged on one side of the discharge vessel 13 B. Here, as an example, it is assumed that two discharge vessels 13B are arranged side by side in the Z direction in FIG. 9A.

As shown in FIG. 9A, the first and second electrodes 14B and 15B are arranged on the side of the discharge vessel 13B (-X direction face) opposite to the light extracting (light emission) surface, spaced apart from each other in the tube axis direction (Y direction) of the discharge vessel 13.

The discharge vessel 13B is arranged such that the discharge vessel 13B straddles those two electrodes 14B and 15B while contacting them. More particularly, the two electrodes 14B and 15B each have a concave groove extending in the Y direction, respectively, and the discharge vessel 13B is fitted into the concave grooves of the electrodes 14B and 15B.

The first electrode 14B and the second electrode 15B are electrically connected to the high-frequency power supply 16A via the power feed line 16b, respectively. By applying a high-frequency AC voltage between the first and second electrodes 14B and 15B, excited dimers are produced in the inner space of the discharge vessel 13B, and excimer light is emitted from the light extraction face (+X direction face) of the excimer lamp 12B.

Here, the electrodes 14B and 15B may be made of metal material that is reflective to the light emitted from the excimer lamp 12B. In this case, light emitted from the discharge vessel 13B in the -X direction can be reflected to travel in the +X direction. The electrodes 14B and 15B can be made of, for example, aluminum (Al) or stainless steel.

Excimer lamps may generate high-frequency noise as high-frequency power is applied thereto to perform high-frequency lighting, as described above. However, by constructing the housing 11 that houses the excimer lamp with conductive metal as described above, it makes it possible to suppress the high frequency noise from being transmitted from the excimer lamp to the outside of the housing 11. Thus, it makes it possible to prevent control commands to other control systems installed near the UV irradiation unit 10 from being disturbed by the high-frequency noise so as to prevent defects in the control commands.

As described above, for the excimer lamp as the UV light source of the inactivation apparatus 100 according to the present embodiment, it is preferable to use a KrCI excimer lamp that emits UV light having a peak wavelength at 222 nm or a KrBr excimer lamp that emits UV light having a peak wavelength at 207 nm.

The UV light having the wavelength of 222 nm, which is emitted from the KrCl excimer lamp, and the UV light having the wavelength of 207 nm, which is emitted from the KrBr excimer lamp, are both safe for humans and animals, and are capable of sterilizing microorganisms and inactivating viruses. Therefore, even when humans or animals are present in a region to be sterilized or inactivated in a space, it makes it possible to perform the sterilization or inactivation operation using UV irradiation.

Although a certain case in which the excimer lamp is used as the UV light source is described in the above embodiment, alternatively, LEDs can also be used as the UV light source.

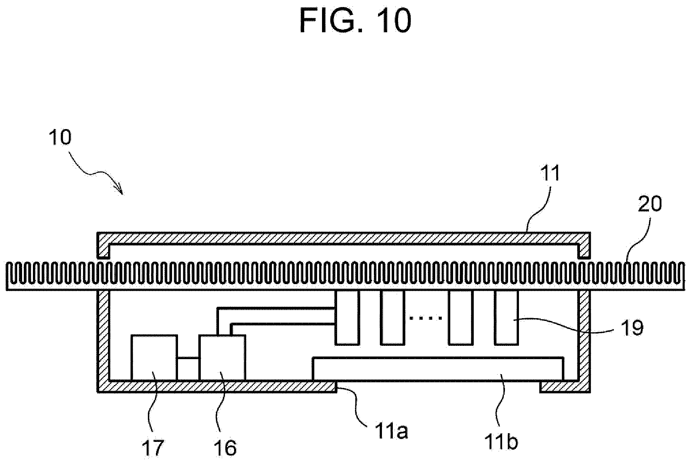

FIG.10 is a schematic diagram exemplarily illustrating a UV irradiation unit 10 using an LED 19 as the UV light source. Referring to FIG. 10, the UV irradiation unit 10 is equipped with a plurality of LEDs 19.

As described above, the wavelength range of UV light used in decontamination (sterilization) applications is 200 nm to 320 nm, and the most effective wavelength is near 260 nm, where nucleic acids (DNA, RNA) have high absorption.

For this reason, for the LEDs 19 as the UV light source mounted in the UV irradiation unit 10, LEDs having the wavelength of 200 nm to 320 nm are employed. More particularly, for example, aluminum gallium nitride (AlGaN) based LED, aluminum nitride (AIN) based LED, and the like, may be employed. The AlGaN based LEDs are capable of producing deep UV (DUV) light in the wavelength range of 200 nm to 350 nm by changing the composition of aluminum (Al). Likewise, the AIN based LEDs emit UV light having a peak wavelength of 210 nm.

It is preferable to adjust the composition of aluminum (Al) for AlGaN based LEDs such that the center wavelength of the LEDs is to be within the range of 200 nm to 237 nm. As described above, UV light within this wavelength range is safe for humans and animals and thus can be used to appropriately sterilize microorganisms and inactivate viruses. For example, by adjusting the composition of Al, it is possible to make an AlGaN based LEDs having the center wavelength of 222 nm.

Alternatively, magnesium zinc oxide (MgZnO) based LEDs can also be used for the LEDs as the UV light source. By changing the composition of magnesium (Mg), MgZnO based LEDs can emit deep UV (DUV) light in the wavelength range of 190 nm to 380 nm.

It is preferable to adjust the composition of Mg for MgZnO based LEDs such that the center wavelength is to be within the range of 200 nm to 237 nm.

As described above, UV light within this wavelength range is safe for humans and animals and thus can be used to appropriately sterilize microorganisms and inactivate viruses. For example, by adjusting the composition of Mg, it is possible to make an MgZnO based LEDs having the center wavelength of 222 nm.

In general, LEDs that emit UV light (in particular, UV light in the deep UV region) as described above have a low luminous efficiency of a few percent or less and generate a large amount of heat. As the heat generation of the LEDs increases, the intensity of the light emitted from the LEDs decreases, and a wavelength shift of the emitted light also occurs. For this reason, in order to suppress the heat rise of the LEDs, as shown in FIG. 10, it is preferable to install LEDs 19 on a cooling member (e.g., a heat-dissipating fin) 20.

At this time, as shown in FIG. 10, a part of the cooling member 20 may protrude from the housing 1 1 of the UV irradiation unit 10. In this case, a part of the cooling member 20 will be exposed to the outside air, and the heat dissipation of the cooling member 20 will proceed more efficiently, so that the heat rise of the LEDs 19 can be appropriately suppressed.

It should be noted that the above AlGaN based LEDs and MgZnO based LEDs, which emit UV light having the center wavelength of 222 nm, also emit UV light in a wavelength range that extends to some extent from the center wavelength of 222 nm. The light emitted from AlGaN based LEDs and MgZnO based LEDs includes a small amount of UV light that is not safe for humans and animals. Therefore, as in the case in which the UV light source is an excimer lamp, it is preferable to use a dielectric multilayer filter (i.e., optical filter) that cuts off light in the UV-C wavelength range with wavelengths other than 200 nm to 237 nm.

The above optical filter is more preferably one that cuts off light in the UV-C wavelength range with wavelengths other than 200 nm to 235 nm, and more preferably one that cuts off light in the UV-C wavelength range with wavelengths other than 200 to 230 nm. This is also true when the light source is an excimer lamp.

On the other hand, the above AlN based LEDs, which emit UV light having a center wavelength of 210 nm, do not require the optical filter described above.

Regardless of whether the UV light source is the excimer lamp or the LEDs, depending on the irradiance on the light-emitting surface of the UV light source in question, the distance from the UV light source to the surface to be irradiated with the UV light, and other factors, in some cases, the irradiance of the UV light of which wavelength may be unsafe for humans and animals at the surface to be irradiated is within the allowable value or less. In such cases, the UV light source is not required to be provided with the above optical filter.

In the above embodiment, a coherent light source such as an excimer laser or wavelength conversion laser can be used as the UV light source. As the laser light source, for example, a KrCl excimer laser that emits laser light having the center wavelength of 222 nm can be used. For example, a compact, microwave-pumped KrCl excimer laser can be used as the KrCl excimer laser.

According to the present embodiment, it has been newly found for the first time that when the effective irradiance of UV light having the wavelength of 200 nm to 235 nm, which is considered to be light with little adverse effect on human and animal cells, is within a certain irradiance range, there is no adverse effect on the human eyes. It makes it possible to inactivate microorganisms and/or viruses existing in a space where a human is present while ensuring the safety of human eyes by controlling the light source such that the effective incident irradiance of the UV light on human eyes present in the space is within a certain irradiance range.

This addresses Goal 3 of the UN-led Sustainable Development Goals (SDGs): "Ensuring healthy lives and promote welfare for all people of all ages" and contributes significantly to Target 3.3 "By 2030, end the epidemics of AIDS, tuberculosis, malaria and neglected tropical diseases and combat hepatitis, water-borne diseases and other communicable diseases".

Although specific embodiments have been described above, the embodiments described are illustrative only and are not intended to limit the scope of the present invention. The apparatus and method described herein may be embodied in other forms than as described above. In addition, it is also possible to appropriately omit, substitute, or modify the above described embodiments without departing from the scope of the present invention. Embodiments with such omissions, substitutions and modifications fall within the scope of the appended claims and equivalents thereof and also fall within the technical scope of the present invention.

### REFERENCE SIGNS LIST

10: UV Irradiation Unit; 11: Housing; 12: Excimer Lamp; 13: Discharge Vessel; 14: First Electrode; 15: Second Electrode; 16: Power Supply Unit; 17: Controller Unit; 18: Support Member; 19: LED; 20: Cooling Member; 100: Inactivation Apparatus

## Claims

1. An inactivation method of inactivating microorganisms and/or viruses by emitting light, comprising:
emitting, from a light source (12), light having a wavelength range that inactivates microorganisms and/or viruses, the light being emitted from the light source in a space (200) where a human (300) is present and the microorganisms and/or viruses exist, the light being ultraviolet light having a wavelength range of 200 nm to 235 nm;
**characterized in that**
the light source (12) is controlled such that an effective incident irradiance of the ultraviolet light on an eye (500) of the human (300) present in the space (200) is within an irradiance range having an upper limit equal to or less than 3.5 *µ*W/cm².

2. The inactivation method according to claim 1, wherein a reference body height of the human (300) is set to a reference height from a floor surface (202) of the space (200), and wherein the light source is controlled such that the effective incident irradiance of the ultraviolet light on the eye (500) of the human (300) is equal to the effective incident irradiance at the reference height.

3. The inactivation method according to claim 1 or 2, wherein the light source (12) is configured to emit the ultraviolet light from above the human (300) to a floor surface (202) of the space (200), and
a lower limit of the irradiance range is equal to the effective incident irradiance of the ultraviolet light at a height of the eye (500) of the human (300) when the effective incident irradiance of the ultraviolet light at the floor surface (202) is the minimum irradiance required for inactivation at the floor surface (202).

4. The inactivation method according to any one of claims 1 to 3, wherein the effective incident irradiance of the ultraviolet light on the eye (500) of the human (300) is an irradiance calculated based on an incident angle of the ultraviolet light from the light source (12) to the eye (500) of the human (300).

5. An inactivation apparatus (100) for carrying out the method according to any one of claims 1 to 4, comprising:
a light irradiation unit (10) including a light source (12) configured to emit light having a wavelength range that inactivates microorganisms and/or viruses, the light source emitting the light in a space (200) where a human (300) is present and the microorganisms and/or viruses exists; and the light source (12) being arranged in the space (200) such that the ultraviolet light is emitted from above the human (300) to a floor surface (202) of the space;
a controller unit configured to control irradiation of the light by the light source,
the light being ultraviolet light having a wavelength range of 200 nm to 235 nm,
**characterized in that**
the controller unit is configured to control the light source (12) to limit an effective incident irradiance of the ultraviolet light on an eye (500) of the human (300) present in the space (200) to be 3.5 *µ*W/cm² or less
and **in that** a reference body height of the human (300) is set to a reference height from the floor surface (202), and the controller unit is configured to control the light source (12) such that the effective incident irradiance of the ultraviolet light on the eye (500) of the human (300) is equal to the effective incident irradiance at the reference height.

6. The inactivation method according to any one of claims 1 to 4 or the inactivation apparatus (100) according to claim 5, wherein
the irradiance range has a lower limit equal to or greater than 1 *µ*W/cm².

7. The inactivation apparatus (100) according to claims 5 or 6, wherein
a lower limit of the irradiance range is equal to the effective incident irradiance of the ultraviolet light at a height of the eye (500) of the human (300) when the effective incident irradiance of the ultraviolet light at the floor surface (202) is the minimum irradiance required for inactivation at the floor surface (202).

8. The inactivation method according to any one of claims 1 to 4 or the inactivation apparatus (100) according to any one of claims 5 to 7, wherein
the controller unit controls the light source (12) such that an irradiation amount of the ultraviolet light irradiated onto the human (300) in the space (200) is between 7 mJ/cm² and 150 mJ/cm² for 12 hours per day.

9. The inactivation apparatus (100) according to any one of claims 5 to 8, wherein
the effective incident irradiance of the ultraviolet light on the eye (500) of the human (300) is an irradiance calculated based on an incident angle of the ultraviolet light from the light source (12) to the eye (500) of the human (300).

10. The inactivation method according to any one of claims 1 to 4 or the inactivation apparatus (100) according to any one of claims 5 to 9, wherein
the light source (12) is at least any one of an excimer lamp, an LED, and a coherent light source.

11. The inactivation method according to any one of claims 1 to 4 or the inactivation apparatus (100) according to any one of claims 5 to 10, wherein
the light source (12) emits ultraviolet light having a center wavelength of 222 nm.

12. The inactivation method according to any one of claims 1 to 4 or the inactivation apparatus (100) according to any one of claims 5 to 10, wherein
the light source is an LED, and
the LED is at least any one of an aluminum gallium nitride (AlGaN) based LED, an aluminum nitride (AlN) based LED, and a magnesium zinc oxide (MgZnO) based LED.

13. The inactivation method according to any one of claims 1 to 4 or the inactivation apparatus (100) according to any one of claims 5 to 10 and 12, wherein
the light source is an LED, and
the light irradiation unit includes a cooling member to cool the LED.

14. The inactivation method according to any one of claims 1 to 4 or the inactivation apparatus (100) according to any one of claims 5 to 11, wherein
the light source is an excimer lamp (12, 12A, 12B), and
the light irradiation unit includes a housing (11) made of conductive metal and configured to house the excimer lamp.

15. The inactivation method according to any one of claims 1 to 4 or the inactivation apparatus (100) according to any one of claims 5 to 11 and 14, wherein
the light irradiation unit (10) includes a housing (11) configured to house the excimer lamp and equipped with a light emission window emitting at least a part of the light emitted from the light source, and
the light emission window is equipped with an optical filter configured to block light in a wavelength range other than 200 nm to 235 nm to pass through the optical filter.

## Patentansprüche

1. Inaktivierungsverfahren zur Inaktivierung von Mikroorganismen und/oder Viren durch Emission von Licht, umfassend:
Emittieren von Licht aus einer Lichtquelle (12) mit einem Wellenlängenbereich, der Mikroorganismen und/oder Viren inaktiviert, wobei das Licht aus der Lichtquelle in einen Raum (200) emittiert wird, in dem ein Mensch (300) anwesend ist und die Mikroorganismen und/oder Viren vorhanden sind, wobei das Licht ultraviolettes Licht mit einem Wellenlängenbereich von 200 nm bis 235 nm ist,
**dadurch gekennzeichnet, dass**
die Lichtquelle (12) so gesteuert wird, dass eine effektive einfallende Bestrahlungsstärke des ultravioletten Lichts auf ein Auge (500) des im Raum (200) anwesenden Menschen (300) sich in einem Bestrahlungsstärkenbereich befindet, der eine Obergrenze von 3,5 µW/cm² oder weniger aufweist.

2. Inaktivierungsverfahren nach Anspruch 1, wobei eine Referenzkörpergröße des Menschen (300) auf eine Referenzhöhe von einer Bodenoberfläche (202) des Raums (200) eingestellt ist, und wobei die Lichtquelle so gesteuert wird, dass die effektive einfallende Bestrahlungsstärke des ultravioletten Lichts auf das Auge (500) des Menschen (300) gleich der effektiven einfallenden Bestrahlungsstärke in der Referenzhöhe ist.

3. Inaktivierungsverfahren nach Anspruch 1 oder 2, wobei die Lichtquelle (12) so ausgelegt ist, dass sie das ultraviolette Licht von oberhalb des Menschen (300) auf eine Bodenoberfläche (202) des Raums (200) emittiert, und
eine Untergrenze des Bestrahlungsstärkenbereichs gleich der effektiven einfallenden Bestrahlungsstärke des ultravioletten Lichts in einer Höhe des Auges (500) des Menschen (300) ist, wenn die effektive einfallende Bestrahlungsstärke des ultravioletten Lichts an der Bodenoberfläche (202) der für die Inaktivierung an der Bodenoberfläche (202) erforderlichen Mindestbestrahlungsstärke entspricht.

4. Inaktivierungsverfahren nach einem der Ansprüche 1 bis 3, wobei die effektive einfallende Bestrahlungsstärke des ultravioletten Lichts auf das Auge (500) des Menschen (300) eine Bestrahlungsstärke ist, die auf der Grundlage eines Einfallswinkels des ultravioletten Lichts von der Lichtquelle (12) auf das Auge (500) des Menschen (300) berechnet wird.

5. Inaktivierungsvorrichtung (100) zur Durchführung des Verfahrens nach einem der Ansprüche 1 bis 4, umfassend:
eine Lichtbestrahlungseinheit (10) mit einer Lichtquelle (12), die so ausgelegt ist, dass sie Licht mit einem Wellenlängenbereich emittiert, der Mikroorganismen und/oder Viren inaktiviert, wobei die Lichtquelle das Licht in einen Raum (200) emittiert, in dem ein Mensch (300) anwesend ist und die Mikroorganismen und/oder Viren vorhanden sind, und wobei die Lichtquelle (12) im Raum (200) so angeordnet ist, dass das ultraviolette Licht von oberhalb des Menschen (300) auf eine Bodenoberfläche (202) des Raums emittiert wird,
eine Steuereinheit, die dazu ausgelegt ist, die Abstrahlung des Lichts durch die Lichtquelle zu steuern,
wobei das Licht ultraviolettes Licht mit einem Wellenlängenbereich von 200 nm bis 235 nm ist,
**dadurch gekennzeichnet, dass**
die Steuereinheit dazu ausgelegt ist, die Lichtquelle so zu steuern, dass eine wirksame einfallende Bestrahlungsstärke des ultravioletten Lichts auf ein Auge (500) des im Raum (200) anwesenden Menschen (300) auf 3,5 µW/cm² oder weniger begrenzt wird,
und dass eine Referenzkörpergröße des Menschen (300) auf eine Referenzhöhe von einer Bodenoberfläche (202) eingestellt ist, und die Steuereinheit dazu ausgelegt ist, die Lichtquelle (12) so zu steuern, dass die effektive einfallende Bestrahlungsstärke des ultravioletten Lichts auf das Auge (500) des Menschen (300) gleich der effektiven einfallenden Bestrahlungsstärke in der Referenzhöhe ist.

6. Inaktivierungsverfahren nach einem der Ansprüche 1 bis 4 oder Inaktivierungsvorrichtung (100) nach Anspruch 5, wobei
der Bestrahlungsstärkenbereich eine Untergrenze von 1 µW/cm² oder mehr aufweist.

7. Inaktivierungsvorrichtung (100) nach Anspruch 5 oder 6, wobei
eine Untergrenze des Bestrahlungsstärkenbereichs gleich der effektiven einfallenden Bestrahlungsstärke des ultravioletten Lichts in einer Höhe des Auges (500) des Menschen (300) ist, wenn die effektive einfallende Bestrahlungsstärke des ultravioletten Lichts an der Bodenoberfläche (202) der für die Inaktivierung an der Bodenoberfläche (202) erforderlichen Mindestbestrahlungsstärke entspricht.

8. Inaktivierungsverfahren nach einem der Ansprüche 1 bis 4 oder Inaktivierungsvorrichtung (100) nach einem der Ansprüche 5 bis 7, wobei
die Steuereinheit die Lichtquelle (12) so steuert, dass die Strahlungsmenge des auf den Menschen (300) im Raum (200) ausgestrahlten ultravioletten Lichts für 12 Stunden pro Tag zwischen 7 mJ/cm² und 150 mJ/cm² beträgt.

9. Inaktivierungsvorrichtung (100) nach einem der Ansprüche 5 bis 8, wobei
die effektive einfallende Bestrahlungsstärke des ultravioletten Lichts auf das Auge (500) des Menschen (300) eine Bestrahlungsstärke ist, die auf der Grundlage eines Einfallswinkels des ultravioletten Lichts von der Lichtquelle (12) auf das Auge (500) des Menschen (300) berechnet wird.

10. Inaktivierungsverfahren nach einem der Ansprüche 1 bis 4 oder Inaktivierungsvorrichtung (100) nach einem der Ansprüche 5 bis 9, wobei
die Lichtquelle (12) mindestens eine einer Excimerlampe, einer LED oder einer kohärenten Lichtquelle ist.

11. Inaktivierungsverfahren nach einem der Ansprüche 1 bis 4 oder Inaktivierungsvorrichtung (100) nach einem der Ansprüche 5 bis 10, wobei
die Lichtquelle ultraviolettes Licht mit einer Zentralwellenlänge von 222 nm emittiert.

12. Inaktivierungsverfahren nach einem der Ansprüche 1 bis 4 oder Inaktivierungsvorrichtung (100) nach einem der Ansprüche 5 bis 10, wobei
die Lichtquelle eine LED ist und
die LED mindestens eine einer LED auf der Basis von Aluminiumgalliumnitrid (AlGaN), einer LED auf der Basis von Aluminiumnitrid (AIN) oder einer LED auf der Basis von Magnesiumzinkoxid (MgZnO) ist.

13. Inaktivierungsverfahren nach einem der Ansprüche 1 bis 4 oder Inaktivierungsvorrichtung (100) nach einem der Ansprüche 5 bis 10 und 12, wobei
die Lichtquelle eine LED ist und
die Lichtabstrahlungseinheit ein Kühlelement zum Kühlen der LED umfasst.

14. Inaktivierungsverfahren nach einem der Ansprüche 1 bis 4 oder Inaktivierungsvorrichtung (100) nach einem der Ansprüche 5 bis 11, wobei
die Lichtquelle eine Excimerlampe (12, 12A, 12B) ist und
die Lichtabstrahlungseinheit ein Gehäuse (11) beinhaltet, das aus leitfähigem Metall besteht und so ausgelegt ist, dass es die Excimerlampe aufnimmt.

15. Inaktivierungsverfahren nach einem der Ansprüche 1 bis 4 oder Inaktivierungsvorrichtung (100) nach einem der Ansprüche 5 bis 11 und 14, wobei
die Lichtbestrahlungseinheit (10) ein Gehäuse (11) beinhaltet, das so ausgelegt ist, dass es die Excimerlampe aufnimmt und mit einem Lichtaustrittsfenster ausgestattet ist, das zumindest einen Teil des von der Lichtquelle emittierten Lichts emittiert, und
das Lichtaustrittsfenster mit einem optischen Filter ausgestattet ist, der so ausgelegt ist, dass er Licht in einem anderen Wellenlängenbereich als 200 nm bis 235 nm daran hindert, den optischen Filter zu passieren.

## Revendications

1. Procédé d'inactivation pour inactiver des microorganismes et/ou des virus par émission de lumière, comprenant :
l'émission à partir d'une source de lumière (12), d'une lumière ayant une gamme de longueurs d'ondes qui inactive les microorganismes et/ou les virus, la lumière étant émise à partir de la source de lumière dans un espace (200) où est présent un être humain (300) et où existent les microorganismes et/ou les virus, la lumière étant une lumière ultraviolette ayant une gamme de longueur d'ondes de 200 nm à 235 nm ;
***caractérisé en ce que***
la source de lumière (12) est contrôlée de telle sorte qu'une irradiance incidente effective de la lumière ultraviolette sur l'œil (500) de l'être humain (300) présent dans l'espace (200) soit dans une plage d'irradiance ayant une limite supérieure égale ou inférieure à 3 µW/cm².

2. Procédé d'inactivation selon la revendication 1, dans lequel une hauteur de référence du corps de l'être humain (300) est fixée à une hauteur de référence partant d'une surface du sol (202) de l'espace (200) et dans lequel la source de lumière est contrôlée de telle sorte que l'irradiance incidente effective de la lumière ultraviolette sur l'oeil (500) de l'être humain (300) soit égale à l'irradiance incidente effective à la hauteur de référence.

3. Procédé d'inactivation selon la revendication 1 ou 2, dans lequel la source de lumière (12) est configurée pour émettre la lumière ultraviolette à partir d'au-dessus de l'être humain (300) jusqu'à une surface (202) du sol de l'espace (200), et
une limite inférieure de la plage d'irradiance est égale à l'irradiance incidente effective de la lumière ultraviolette à hauteur de l'oeil de l'être humain (500) lorsque l'irradiance incidente effective de la lumière ultraviolette à la surface du sol (202) est l'irradiance minimum requise pour l'inactivation à la surface du sol (202).

4. Procédé d'inactivation selon l'une quelconque des revendications 1 à 3, dans lequel l'irradiance incidente effective de la lumière ultraviolette sur l'oeil (500) de l'être humain (300) est une irradiance calculée sur la base d'un angle incident de la lumière ultraviolette allant de la source de lumière (12) à l'oeil (500) de l'être humain (300).

5. Dispositif d'inactivation (100) pour mettre en oeuvre le procédé selon l'une quelconque des revendications 1 à 4, comprenant :
une unité d'irradiation lumineuse (10) comprenant une source de lumière (12) configurée pour émettre une lumière ayant une gamme de longueurs d'ondes qui inactive les microorganismes et/ou les virus, la source de lumière émettant une lumière dans un espace (200) où est présent un être humain (300) et où existent les microorganismes et/ou les virus ; et la source de lumière (12) étant disposée dans l'espace (200) de telle sorte que la lumière ultraviolette soit émise depuis au-dessus de l'être humain (300) jusqu'à une surface (202) du sol de l'espace ;
une unité de contrôle configurée pour contrôler l'irradiation lumineuse par la source de lumière,
la lumière étant une lumière ultraviolette ayant une gamme de longueurs d'ondes de 200 nm à 235 nm ;
***caractérisé en ce que***
l'unité de contrôle est configurée pour contrôler la source de lumière (12) afin de limiter une irradiance incidente effective de la lumière ultraviolette sur un oeil (500) de l'être humain (300) présent dans l'espace (200) à 3 µW/cm² ou moins,
et ***en ce qu**'une* hauteur de référence du corps de l'être humain (300) est fixée à une hauteur de référence partant de la surface du sol (202), et l'unité de contrôle est configurée pour commander la source de lumière (12) de telle sorte que l'irradiance incidente effective de la lumière ultraviolette sur l'oeil (500) de l'être humain (300) soit égale à l'irradiance incidente effective à la hauteur de référence.

6. Procédé d'inactivation selon l'une quelconque des revendications 1 à 4 ou dispositif d'inactivation (100) selon la revendication 5, dans lequel
la plage d'irradiance a une limite inférieure égale ou supérieure à 1 µW/cm².

7. Dispositif d'inactivation (100) selon les revendications 5 ou 6, dans lequel
une limite inférieure de la plage d'irradiance est égale à l'irradiance incidente effective de la lumière ultraviolette à hauteur de l'oeil (500) de l'être humain (300) lorsque l'irradiance incidente effective de la lumière ultraviolette à la surface du sol (202) est l'irradiance minimum requise pour l'inactivation à la surface du sol (202).

8. Procédé d'inactivation selon l'une quelconque des revendications 1 à 4 ou dispositif d'inactivation (100) selon l'une quelconque des revendications 5 à 7, dans lequel
l'unité de contrôle contrôle la source de lumière (12) de telle sorte qu'une quantité d'irradiation de la lumière ultraviolette irradiée sur l'être humain (300) dans l'espace (200) soit comprise entre 7 mJ/cm² et 150 mJ/cm² pendant 12 heures par jour.

9. Dispositif d'inactivation (100) selon l'une quelconque des revendications 5 à 8, dans lequel
l'irradiance incidente effective de la lumière ultraviolette sur l'oeil (500) de l'être humain (300) est une irradiance calculée sur la base d'un angle incident de la lumière ultraviolette de la source de lumière (12) à l'oeil (500) de l'être humain (300).

10. Procédé d'inactivation selon l'une quelconque des revendications 1 à 4 ou dispositif d'inactivation (100) selon l'une quelconque des revendications 5 à 9, dans lequel
la source de lumière (12) est au moins l'une d'entre une lampe à excimère, une diode électroluminescente (DEL), et une source de lumière cohérente.

11. Procédé d'inactivation selon l'une quelconque des revendications 1 à 4 ou dispositif d'inactivation (100) selon l'une quelconque des revendications 5 à 10, dans lequel
la source de lumière (12) émet une lumière ultraviolette ayant une longueur d'onde centrale de 222 nm.

12. Procédé d'inactivation selon l'une quelconque des revendications 1 à 4 ou dispositif d'inactivation (100) selon l'une quelconque des revendications 5 à 10, dans lequel
la source de lumière est une diode électroluminescente (DEL), et
la diode électroluminescente (DEL) est au moins l'une d'entre une DEL à base de nitrure d'aluminium-gallium (AlGaN), une DEL à base de nitrure d'aluminium (AlN), et une DEL à base d'un oxyde de magnésium et zinc (MgZnO).

13. Procédé d'inactivation selon l'une quelconque des revendications 1 à 4 ou dispositif d'inactivation (100) selon l'une quelconque des revendications 5 à 10 et 12, dans lequel
la source de lumière est une diode électroluminescente (DEL), et
l'unité d'irradiation lumineuse comprend un élément de refroidissement pour refroidir la diode électroluminescente.

14. Procédé d'inactivation selon l'une quelconque des revendications 1 à 4 ou dispositif d'inactivation (100) selon l'une quelconque des revendications 5 à 11, dans lequel
la source de lumière est une lampe à excimère (12, 12A, 12B), et
l'unité d'irradiation lumineuse comprend un boîtier (11) fait d'un matériau conducteur et configuré pour recevoir la lampe à excimère.

15. Procédé d'inactivation selon l'une quelconque des revendications 1 à 4 ou dispositif d'inactivation (100) selon l'une quelconque des revendications 5 à 11 et 14, dans lequel
l'unité d'irradiation lumineuse (10) comprend un boîtier (11) configuré pour recevoir la lampe à excimère et équipé d'une fenêtre d'émission de la lumière émettant au moins une partie de la lumière émise à partir de la source de lumière, et
la fenêtre d'émission de la lumière est équipée d'un filtre optique configuré pour empêcher que la lumière dans une plage de longueurs d'ondes autres que de 200 nm à 235 nm ne traverse le filtre optique.
